(19) Europäisches Patentamt
European Patent Office
Office européen des brevets

(11) EP 4 659 587 A1

(12) **EUROPEAN PATENT APPLICATION**
published in accordance with Art. 153(4) EPC

(43) Date of publication:
**10.12.2025 Bulletin 2025/50**

(21) Application number: **24852314.4**

(22) Date of filing: **07.08.2024**

(51) International Patent Classification (IPC):
***A23K 20/105*** *(2016.01)* ***A61K 31/19*** *(2006.01)*
***A61P 33/02*** *(2006.01)* ***A01N 37/02*** *(2006.01)*
***A01P 1/00*** *(2006.01)* ***C11D 3/20*** *(2006.01)*

(52) Cooperative Patent Classification (CPC):
**A01N 37/02; A01P 1/00; A23K 20/105; A61K 31/19; A61P 33/02; C11D 3/20**

(86) International application number:
**PCT/KR2024/011650**

(87) International publication number:
**WO 2025/033960 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.08.2023 KR 20230103638**

(71) Applicant: **CJ CheilJedang Corporation**
**Seoul 04560 (KR)**

(72) Inventors:
- **SON, Kyuyeol**
  **Seoul 04560 (KR)**
- **LEE, Kyung Min**
  **Seoul 04560 (KR)**
- **MOON, Jun Ok**
  **Seoul 04560 (KR)**
- **CHAE, Jong Pyo**
  **Seoul 04560 (KR)**
- **PARK, Si-Nae**
  **Seoul 04560 (KR)**

(74) Representative: **Meissner Bolte Partnerschaft mbB**
**Patentanwälte Rechtsanwälte**
**Postfach 86 06 24**
**81633 München (DE)**

(54) **ANTI-COCCIDIAL COMPOSITION COMPRISING QUINIC ACID AND USES THEREOF**

(57) The present disclosure relates to a composition comprising quinic acid for preventing, alleviating, or treating coccidiosis, and an anti-protozoan composition against sporozoites of the genus Eimeria.

【FIG. 1】

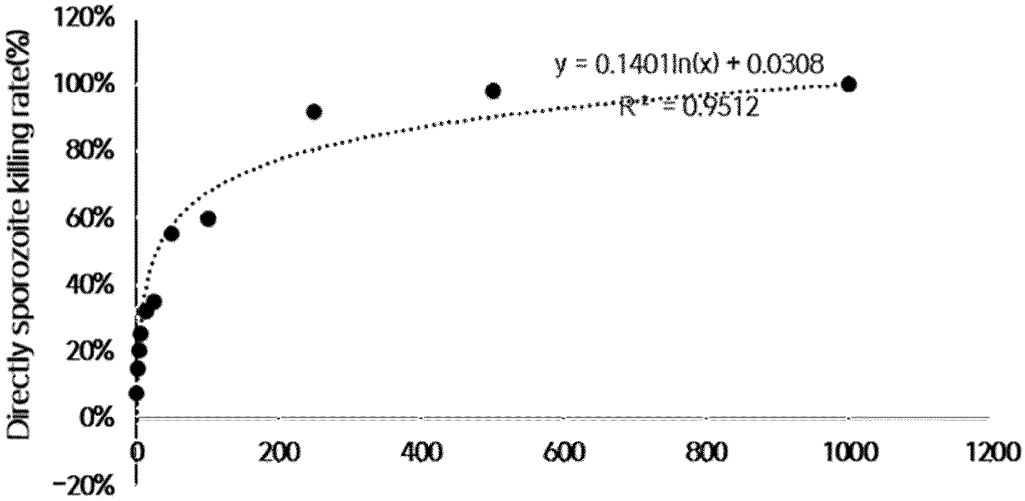




Processed by Luminess, 75001 PARIS (FR)

## Description

[TECHNICAL FIELD]

Cross-reference to related application(s)

The present application claims the benefit of the priority based on Korean Patent Application No. 10-2023-0103638 filed on August 8, 2023, and the entire contents disclosed in the document of the corresponding Korean patent application are incorporated as a part of the present description.

The present disclosure relates to an anti-coccidial use of quinic acid and/or its salt.

[BACKGROUND ART]

Coccidiosis is an intestine-related disease caused by a protozoal parasite belonging to the phylum apicomplexan called *Eimeria,* and infection with coccidiosis shows symptoms such as digestive problems, diarrhea, and weight loss, and much further, causes livestock mortality, and has an economically profound effect (Williams RB. A compartmentalised model for the estimation of the cost of coccidiosis to the world's chicken production industry. Int J Parasitol. 1999; 29(8):1209-1229).

Over the past few years, many researchers have developed anti-coccidial agents such as ionophores or chemically synthetic compounds which can prevent formation of oocyst cell walls or asexual and sexual reproduction of protozoa as a therapeutic agent for treating coccidiosis. However, side effects such as emergence of drug-resistant protozoa and the like due to long-term use of the shuttle program which alternatively treats ionophores and chemically synthetic compounds occurred.

In particular, antibiotics accumulated in animals due to abuse and misuse of antibiotics become a serious problem as humans ingest antibiotics through meat, and due to the problem of residue of antibiotics in livestock products, there is a trend that all countries around the world are banning the administration of antibiotics. Therefore, there is an urgent need to develop and research an alternative to conventional anti-coccidial agents showing side effects such as problems of emergence of drug-resistant strains and residue in the body and the like.

[PRIOR ART]

(Patent document 1) U.S. Pante Publication US 2003-0091589 A1

[DISCLOSURE]

[TECHNICAL PROBLEM]

An object of the present disclosure is to provide a use for using in prevention, improvement, and/or treatment of coccidiosis of quinic acid and/or its salt.

Another object of the present disclosure is to provide a feed composition for preventing or alleviating coccidiosis comprising quinic acid and/or its salt.

Other object of the present disclosure is to provide a pharmaceutical composition for preventing or treating coccidiosis comprising quinic acid and/or its salt.

Other object of the present disclosure is to provide a use of quinic acid and/or its salt for using in preparation of a composition (feed composition, pharmaceutical composition) for preventing, alleviating and/or treating coccidiosis.

Other object of the present disclosure is to provide a method for preventing, alleviating, and/or treating coccidiosis, comprising administering quinic acid and/or its salt into a subject in need of preventing, alleviating and/or treating coccidiosis. The method may further comprise confirming a subject in need of preventing, alleviating and/or treating coccidiosis, before the administering.

Other object of the present disclosure is to provide an anti-protozoal use against an *Eimeria* sp. protozoan of quinic acid and/or its salt.

Other object of the present disclosure is to provide an anti-protozoal composition against an *Eimeria* sp. protozoan comprising quinic acid and/or its salt.

Other object of the present disclosure is to provide a use of quinic acid and/or its salt for using in preparation of an anti-protozoal composition against an *Eimeria* sp. protozoan.

Other object of the present disclosure is to provide a method for controlling an *Eimeria* sp. protozoan, comprising administering quinic acid and/or its salt into a subject in need of control of an *Eimeria* sp. protozoan.

[TECHNICAL SOLUTION]

This is explained in detail as follows. On the other hand, each description and embodiment disclosed in the present disclosure can also be applied to each other description and embodiment. In other words, all combinations of the various elements disclosed in the present disclosure belong to the scope of the present disclosure. In addition, the scope of the present disclosure cannot be considered limited by the specific description described below. Furthermore, those skilled in the art can recognize or confirm many equivalents to the specific embodiments of the present disclosure described in the present disclosure using only a common experiment. In addition, these equivalents are intended to be incorporated in the present disclosure.

Hereinafter, the present invention will be described in more detail.

One aspect provides a composition for preventing, alleviating, and/or treating coccidiosis comprising quinic acid and/or its salt.

Another aspect provides a use of quinic acid and/or its salt for using in preparation of a composition for preventing, alleviating and/or treating coccidiosis.

Other aspect provides a method for preventing, alleviating, and/or treating coccidiosis, comprising administering quinic acid and/or tis salt into a subject in need of preventing, alleviating, and/or treating coccidiosis. The method may further comprise confirming a subject in need of preventing, alleviating, and/or treating coccidiosis, before the administering. According to one embodiment, the confirming a subject may comprise detecting oocysts of protozoa capable of inducing coccidiosis in feces separated from the subject.

The composition for preventing, alleviating, and/or treating coccidiosis may be a feed composition or a pharmaceutical composition.

In the present disclosure, quinic acid may be a compound having the structure of the following Chemical formula 1 ($C_7H_{12}O_6$, Cas No. 77-95-2, PubChem CID NO. 6508).

[Chemical formula 1]

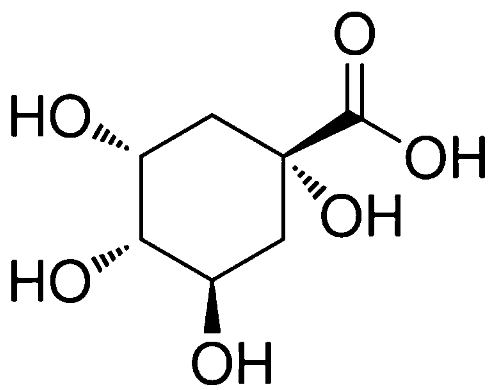

The quinic acid or its salt may be purchased commercially, or be obtained by being extracted and separated from a natural product or a strain, or be prepared by a common organic synthesis method, but not limited thereto.

In the present description, the salt of quinic acid may mean a physiologically acceptable salt of salts that are substances in which a cation and an anion are bound by electrostatic interaction, and for example, it may mean a salt acceptable for a feed composition, a pharmaceutically acceptable salt, and/or a salt acceptable for an anti-protozoal composition. For example, the salt may be at least one selected from the group consisting of metal salts, salts with organic bases, salts with inorganic acids, salts with organic acids, salts with basic or acidic amino acids, and the like. In one embodiment, the metal salts may be at least one selected from the group consisting of alkali metal salts (sodium salts, potassium salts, etc.), alkali earth metal salts (calcium salts, magnesium salts, barium salts, etc.), aluminum salts and the like; and the salts with organic bases may be at least one selected from the group consisting of salts with triethylamine, pyridine, picoline, 2,6-lutidine, ethanolamine, diethanolamine, cyclohexylamine, dicyclohexylamine, N,N-dibenzylethyldiamine, and the like; and the salts with inorganic acids may be at least one selected from the group consisting of salts with hydrochloric acid, hydrobromic acid, nitric acid, sulfuric acid, phosphoric acid and the like; and the salts with organic acids may be at least one selected from the group consisting of salts with formic acid, acetic acid, trifluoroacetic acid, phthalic acid, fumaric acid, oxalic acid, tartaric acid, maleic acid, citric acid, succinic acid, methane sulfonic acid, benzene sulfonic acid, p-toluene sulfonic acid, and the like; and the salts with basic amino acids may be at least one selected from the group consisting of salts with arginine, lysine, ornithine and the like; and the salts with acidic amino acids may be at least one selected from the group consisting of salts with aspartic acid, glutamic acid and the like.

In the present disclosure, that the anti-coccidial efficacy (activity, effect) is excellent may refer to at least one (for example, 1 kind or more, 2 kinds or more, 3 kinds or more, or all 4 kinds) selected from the group consisting of the following (1) to (4):

(1) An anticoccidial index (ACI) is higher than a control group;
(2) A mortality rate, a lesion score (for example, cecal lesion score) and/or a released amount of fecal oocysts are reduced compared to a control group by being administered into a coccidiosis-induced animal subject;
(3) Body loss by induction of coccidiosis is inhibited; and
(4) Insecticidal activity against protozoa inducing coccidiosis is higher than a control group.

In the present disclosure, the control group may mean a negative control group (a nothing-treated group or a water and/or buffer-treated group) and/or a positive control group comprising a conventionally known anti-coccidial agent (for example, diclazuril and/or salinomycin).
The composition according to one embodiment may have at least one (for example, 1 kind or more, 2 kinds or more, 3 kinds or more, 4 kinds or more, 5 kinds or more, or all 6 kinds) characteristic selected from the group consisting of the following (1) to (6), and the characteristics thereof may be superior to a control group:

(1) excellent anti-coccidial activity;
(2) excellent anti-protozoal effect against protozoa inducing coccidiosis;
(3) excellent acid-resistance;
(4) excellent heat-resistance;
(5) excellent in vivo stability and/or safety; and
(6) excellent weight gain-improving effect.

The composition according to one embodiment can maintain excellent anti-coccidial activity for a long period of time, have excellent stability in vivo, maintain excellent anti-coccidial activity in the environment in various temperature and/or pH ranges, be applied to various products (for example, feed additives), and have excellent storage stability, when administered in the body.
The composition according to one embodiment can have excellent safety in vivo, since it is not absorbed in other tissues and organs (for example, blood, liver, kidney, and/or spleen, etc.) other than intestine when administered in the body, so the amount of residues in the body is small.
In one embodiment, the excellent weight gain-improving effect may mean an excellent effect of increasing the body weight of the subject, by being administered into a subject, and in one embodiment, the weight gain may mean a daily weight gain, and the subject may be a coccidiosis-induced subject.
The composition according to one embodiment may not cause side effects or drug resistance, while exhibiting equivalent or more anti-coccidial activity than conventionally known anti-coccidial agents, and therefore, it may be safe for a long time.
In the present disclosure, "prevention" may refer to all actions of inhibiting or delaying occurrence of disease by administration of the composition according to one embodiment, and "treatment" may mean all actions in which symptoms of suspected and outbreak subjects of disease by administration of the composition according to one embodiment are improved or beneficially changed, and "improvement" may mean all actions of at least reducing parameters related to a condition in which the disease is treated by administration of the composition according to one embodiment, for example, the degree of symptoms. The disease may mean coccidiosis.
In the present disclosure, "coccidiosis" is a disease in which coccidiosis protozoa (protozoa capable of inducing coccidiosis, for example, an *Eimeria* sp. coccidiosis protozoa) are parasitic in the cytoplasm of submucosal tissue in the digestive tract epithelium, and destroy the epithelium and cause enteritis, and is a protozoal disease that causes economic damage by reduction of weight gain and extension of the marketing day due to soft stool, diarrhea and bloody stool in broiler farms. Coccidiosis may be expressed in not only broilers but also birds and mammals, and specifically, the coccidiosis may be infected into cattle, rabbits, goats, dogs, cats, experimental animals, mice and rats, and the like, and in particular, it may cause fatal damage to poultry such as chickens, and the like. In one embodiment, the coccidiosis may include acute coccidiosis, subacute coccidiosis, and chronic coccidiosis and the like. The acute coccidiosis shows bloody stool, loos of energy and anemia within 48 hours after infection, and infected subjects may die, and the subacute coccidiosis may show bloody diarrhea and/or anemia symptoms after infection, and the chronic coccidiosis may show symptoms of soft stool after diarrhea and/or weight loss for 1~2 days after infection.
Oocysts (cysts, eggs) of coccidiosis protozoal species are infectious when they mature into sporulated oocysts at high humidity and temperature, and when oocysts are excreted through feces after passing through a certain life cycle in the body of a subject, transmission occurs easily and the oocyst life cycle is repeated. It is known that oocysts (cysts) of the coccidiosis protozoal species are highly resistant to the external environment, and the cyst wall is composed of inner and

outer two layers. The outer layer of the cyst wall is a gelatin material and strongly resists to physical external pressure, and the inner layer is rich in nuclear proteins and can strongly resist to chemical stimuli, for example, disinfectants. The oocysts of the coccidiosis protozoal species may comprise 4 sporocysts, and each of the sporocysts may comprise 2 sporozoites, and they are infected to an animal in the oocyst form, and then they are excreted in the sporocyst and sporozoite forms, and proliferate in cells, and the sporozoites that have gone through sexual reproduction and/or asexual reproduction may form oocysts and be excreted in feces. In one embodiment, the sporozoites may be used as the same meaning as protozoa, and the sporozoites (protozoa) may cause lesions.

According to one embodiment, the coccidiosis may be induced by *Eimeria* sp. protozoa. In one embodiment, the *Eimeria* sp. protozoa may be at least one selected from the group consisting of *Eimeria acervuline*, *Eimeria tenella*, *Eimeria maxima*, *Eimeria necatrix, Eimeria brunetti, Eimeria hagani, Eimeria mitis, Eimeria praecox, Eimeria mivati, Eimeria aurati, Eimeria baueri, Eimeria lepidosirenis, Eimeria leucisci, Eimeria rutile, Eimeria vanasi, Eimeria amphisbaeniarum*, *Eimeria witchery, Eimeria yemenensae, Eimeria adenoeides, Eimeria colchici, Eimeria curvata, Eimeria dispersa, Eimeria duodenalis, Eimeria fraterculae, Eimeria gallopavonis, Eimeria innocua, Eimeria meleagridis, Eimeria meleagrimitis, Eimeria phasiani, Eimeria procera, Eimeria purpureicephali, Eimeria ahsata, Eimeria alabamensis, Eimeria alijevi, Eimeria aspheronica, Eimeria arloingi, Eimeria arundeli*, *Eimeria bakuensis, Eimeria bovis, Eimeria cameli*, *Eimeria caprina, Eimeria caprovina, Eimeria christenseni, Eimeria clethrionomyis, Eimeria coecicola, Eimeria contorta, Eimeria couesii, Eimeria crandallis*, *Eimeria dammahensis, Eimeria dowleri, Eimeria exigua*, *Eimeria falciformis*, *Eimeria farasanii, Eimeria ferrisi, Eimeria flavescens, Eimeria gallatii, Eimeria granulosa, Eimeria hirci, Eimeria intestinalis, Eimeria irresidua, Eimeria intricata, Eimeria jolchijevi, Eimeria krijgsmanni, Eimeria larimerensis, Eimeria macusaniensis, Eimeria magna, Eimeria marconii, Eimeria media, Eimeria melanuri, Eimeria myoxi, Eimeria nagpurensis, Eimeria nieschulzi, Eimeria ninakohlyakimovae, Eimeria ovinoidalis, Eimeria pallida, Eimeria palustris, Eimeria papillata, Eimeria perforans*, *Eimeria phocae, Eimeria pileata, Eimeria pipistrellus, Eimeria piriformis, Eimeria prionotemni, Eimeria procyonis, Eimeria punctate, Eimeria roobroucki, Eimeria saudiensis, Eimeria sealanderi*, *Eimeria separata, Eimeria stiedae*, *Eimeria ursini*, *Eimeria vermiformis*, *Eimeria weybridgensis*, *Eimeria wobati*, and *Eimeria zuernii.*

The composition according to one embodiment may have an excellent effect of prevention, improvement, and/or treatment of coccidiosis induced by at least one protozoan selected from the group consisting of the *Eimeria* sp. protozoa described in Table 1 below, and the *Eimeria* sp. protozoa described in Table 1 below may induce coccidiosis of an animal described in Table 1, respectively.

[Table 1]

| | species | Host animal |
|---|---|---|
| 1 | *Eimeria acervulina* | Chicken (*Gallus gallus domesticus)* |
| 2 | *Eimeria tenella* | Chicken (*Gallus gallus domesticus)* |
| 3 | *Eimeria maxima* | Chicken (*Gallus gallus domesticus*) |
| 4 | *Eimeria necatrix* | Chicken (*Gallus gallus domesticus*) |
| 5 | *Eimeria brunetti* | Chicken (*Gallus gallus domesticus)* |
| 6 | *Eimeria haqani* | Chicken *(Gallus gallus domesticus)* |
| 7 | *Eimeria mitis* | Chicken (*Gallus gallus domesticus*) |
| 8 | *Eimeria praecox* | Chicken (*Gallus gallus domesticus*) |
| 9 | *Eimeria mivati* | Chicken (*Gallus gallus domesticus*) |
| 10 | *Eimeria aurati* | Goldfish (goldfish (*Carassius auratus*)) |
| 11 | *Eimeria baueri* | Crucian carp (*Carassius carassius*)) |
| 12 | *Eimeria lepidosirenis* | South American lungfish (*Lepidosiren paradoxa*) |
| 13 | *Eimeria leucisci* | Common barbel (*Barbus bocaqei*) |
| 14 | *Eimeria rutile* | European chub (*Leuciscus cephalus cabeda*), Iberian nase (*Chondrostoma polylepis polylepis*) |
| 15 | *Eimeria vanasi* | Blue tilapia (*Oreochromis aureus*) |
| 16 | *Eimeria amphisbaeniarum* | Mann's worm lizard (*Amphisbaena manni)* |
| 17 | *Eimeria witchery* | Mann's worm lizard (*A. manni*) |
| 18 | *Eimeria yemenensae* | Rock agama (*Agama yemenensis*) |

(continued)

| | species | Host animal |
|---|---|---|
| 19 | *Eimeria adenoeides* | Turkey (*Meleagris gallopavo*) |
| 20 | *Eimeria colchici* | Common pheasant (*Phasianus colchicus*) |
| 21 | *Eimeria curvata* | Ruddy ground dove (*Columbina talpacoti*), scaled dove (*Scardafella squammata*) |
| 22 | *Eimeria dispersa* | Turkey (*M. gallopavo*), bobwhite quail (*Colinus virginianus*) |
| 23 | *Eimeria duodenalis* | Common pheasant (*Phasianus colchicus*) |
| 24 | *Eimeria fraterculae* | Atlantic puffin (*Fratercula arctica*) |
| 25 | *Eimeria gallopavonis* | Turkey (*M. qallopavo*) |
| 26 | *Eimeria innocua* | Turkey (*M. gallopavo*) |
| 27 | *Eimeria meleaqridis* | Turkey (*M. gallopavo*) |
| 28 | *Eimeria meleagrimitis* | Turkey (*M. gallopavo)* |
| 29 | *Eimeria phasiani* | Pheasant (*P. colchicus*) |
| 30 | *Eimeria procera* | Grey partridges (*Perdix perdix*) |
| 31 | *Eimeria purpureicephali* | Red-capped parrot (*Purpureicephalus spurius*) |
| 32 | *Eimeria ahsata* | Goat (*Capra hircus*), sheep (*Ovis aries*) |
| 33 | *Eimeria alabamensis* | Cattle (*Bos taurus*) |
| 34 | *Eimeria alijevi* | Goat (*C. hircus)* |
| 35 | *Eimeria aspheronica* | Goat (*C. hircus)* |
| 36 | *Eimeria arloinqi* | Goat (*C. hircus)* |
| 37 | *Eimeria arundeli* | Common wombat (*Vombatus ursinus*) |
| 38 | *Eimeria bakuensis* | Sheep (*O. aries*) |
| 39 | *Eimeria bovis* | Cattle (*B. taurus*) |
| 40 | *Eimeria cameli* | Camels (*Camelus bactrianus, Camelus dromedarius*) |
| 41 | *Eimeria caprina* | Goat (*C. hircus)* |
| 42 | *Eimeria caprovina* | Goat (*C. hircus)* |
| 43 | *Eimeria christenseni* | Goat (*C. hircus)* |
| 44 | *Eimeria clethrionomyis* | Red-backed vole (*Clethrionomys gapperi*) |
| 45 | *Eimeria coecicola* | Rabbit (*Oryctolagus cuniculus*)\ |
| 46 | *Eimeria contorta* | Mouse (*Mus musculus*) |
| 47 | *Eimeria couesii* | Rice rat (*Oryzomys couesi*) |
| 48 | *Eimeria crandallis* | Sheep (*O. aries*) |
| 49 | *Eimeria dammahensis* | Scimitar-homed oryx (*Oryx dammah*) |
| 50 | *Eimeria dowleri* | Eastern red bat (*Lasiurus borealis*) |
| 51 | *Eimeria exiqua* | Rabbit (*O. cuniculus*) |
| 52 | *Eimeria falciformis* | Mouse (*M. musculus*) |
| 53 | *Eimeria farasanii* | Mountain gazelle (*Gazella qazelle farasani*) |
| 54 | *Eimeria ferrisi* | Mouse (*M. musculus*) |
| 55 | *Eimeria flavescens* | Rabbit (*O. cuniculus*) |
| 56 | *Eimeria qallatii* | Red-backed vole (*Clethrionomys qapperi*) |
| 57 | *Eimeria granulosa* | Goat (*C. hircus*) |

(continued)

| | species | Host animal |
|---|---|---|
| 58 | *Eimeria hirci* | Goat (*C. hircus*) |
| 59 | *Eimeria intestinalis* | Rabbit (*O. cuniculus*) |
| 60 | *Eimeria irresidua* | Rabbit (*O. cuniculus*) |
| 61 | *Eimeria intricata* | Goat (*C. hircus)* |
| 62 | *Eimeria jolchijevi* | Goat (*C. hircus)* |
| 63 | *Eimeria krijgsmanni* | Mouse (M. musculus) |
| 64 | *Eimeria larimerensis* | Uinta ground squirrel (*Spermophilus armatus*) |
| 65 | *Eimeria macusaniensis* | llamas (*Lama glama*), guanacos (*Lama guanicoe*), alpacas (*Vicugna pacos*), vicunas (*Vicugna vicugna*) |
| 66 | *Eimeria magna* | Rabbit (*O. cuniculus*) |
| 67 | *Eimeria marconii* | Red-backed vole (*Clethrionomys gapperi*) |
| 68 | *Eimeria media* | Rabbit (*O. cuniculus*) |
| 69 | *Eimeria melanuri* | Garden dormouse (*Eliomys quercinus*) |
| 70 | *Eimeria myoxi* | Garden dormouse (Eliomys quercinus) |
| 71 | *Eimeria nagpurensis* | Rabbit (*O. cuniculus*) |
| 72 | *Eimeria nieschulzi* | Brown rat (*R. norvegicus*) |
| 73 | *Eimeria ninakohlyakimovae* | Goat (*C. hircus)* |
| 74 | *Eimeria ovinoidalis* | Sheep (*O. aries*) |
| 75 | *Eimeria pallida* | Goat (C. hircus) |
| 76 | *Eimeria palustris* | Marsh rice rat (*Oryzomys palustris*) |
| 77 | *Eimeria papillata* | Mouse (*M. musculus*) |
| 78 | *Eimeria perforans* | Rabbit (*O. cuniculus*) |
| 79 | *Eimeria phocae* | Sable Island harbour seals (*Phoca vitulina*) |
| 80 | *Eimeria pileata* | Red-backed vole (*Clethrionomys gapperi*) |
| 81 | *Eimeria pipistrellus* | Kuhl’s pipistrelle (*Pipistrellus kuhlii*) |
| 82 | *Eimeria piriformis* | Rabbit (*O. cuniculus)* |
| 83 | *Eimeria prionotemni* | Bennett’s wallaby (*Macropus rufoqriseus*) |
| 84 | *Eimeria procyonis* | Raccoon (*Procyon lotor*) |
| 85 | *Eimeria punctate* | Goat (*C. hircus*) |
| 86 | *Eimeria roobroucki* | Rabbit (*O. cuniculus*) |
| 87 | *Eimeria saudiensis* | Arabian oryx (*Oryx leucoryx*) |
| 88 | *Eimeria sealanderi* | Eastern red bat (*Lasiurus borealis*) |
| 89 | *Eimeria separata* | Mouse (*M. musculus*), rat (*Rattus rattus*) |
| 90 | *Eimeria stiedae* | Rabbit (*O. cuniculus*) |
| 91 | *Eimeria ursini* | Southern hairy nosed wombat (*Lasiorhinus latifrons*) |
| 92 | *Eimeria vermiformis* | Mouse (*M. musculus*) |
| 93 | *Eimeria weybridgensis* | Sheep (*O. aries*) |
| 94 | *Eimeria wobati* | Southern hairy-nosed wombat (*L. latifrons*) |
| 95 | *Eimeria zuernii* | Cattle (*B. taurus*) |

The composition according to one embodiment may have excellent effects of preventing, alleviating, and/or treating coccidiosis induced by *Eimeria tenella*.

In one embodiment, the preventing or alleviating coccidiosis may refer to at least one (for example, 1 or more kinds, 2 or more kinds, 3 or more kinds, or all 4 kinds) selected from the group consisting of the following (1) to (3), and for example, it may be that at least one (for example, 1 or more kinds, 2 or more kinds, or all 3 kinds) selected from the group consisting of the following (1) to (4) is reduced, inhibited, and/or increased compared to a control group (negative control group and/or positive control group):

(1) Reduction of at least one selected from the group consisting of a lesion score (for example, cecal lesion score), a released amount of fecal oocysts, and a mortality rate;
(2) Inhibition of weight loss by coccidiosis; and
(3) An increase of an anticoccidial index (ACI).

In one embodiment, a lesion scoring method for determining the lesion score may be performed with reference to Johnson JK & Reid WM (1970) document (Joyce Johnson, W.Malcolm Reid, Anticoccidial drugs: Lesion scoring techniques in battery and floor-pen experiments with chickens, Experimental parasitology, 1970), and the lesion score may be 0 to 4 classes. In one embodiment, the lesion score may mean a lesion score measured in the cecum, duodenum, and/or jejunum, and it may be calculated by the sum of each of the lesion scores measured in each organ (cecum, duodenum, and/or jejunum).

In one embodiment, the released amount of the fecal oocysts may be measured by using a microscope or using a counting chamber (for example, McMaster chamber) and the like by collecting feces released from a subject.

In one embodiment, the mortality rate may mean a mortality rate of an animal subject in which coccidiosis is induced, and by performing a post mortem, the number of subjects that died from causes other than coccidiosis may be excluded.

In one embodiment, the coccidiosis-induced subjects may have a body weight reduced than subjects in which coccidiosis is not induced, and the composition according to one embodiment may inhibit a weight loss by induction of coccidiosis.

In one embodiment, the anticoccidial index may be calculated by the following Equation 1, and in Equation 1, the lesion score may be calculated by the afore-mentioned method.

Anticoccidial index (ACI) = (Survival rate after challenge (%)) + (Weight gain per day compared to negative control group (%)) - (Lesion score × 10) - (Index of released amount of fecal oocysts) (Equation 1)

The challenge may mean administration (for example, oral inoculation, etc.) of protozoa capable of inducing coccidiosis. In one embodiment, the survival rate may be a survival rate measured on days 5 to 10, days 7 to 10, days 8 to 10, days 7 to 9, days 7 to 8, or day 7 after challenge, and the survival rate may be measured by excluding the number of subjects died from causes other than coccidiosis by performing a post mortem.

In the Equation 1, the weight gain compared to the negative control group may a value calculated by a percentage based on a value of the negative control group (for example, negative control group uninfected with protozoa).

In the Equation 1, the lesion score is as same as described above.

In the Equation 1, the index of the released amount of fecal oocysts may be a value of quantifying to 0 when the calculated result value is at a level of 0% or more to less than 1%, 5 when it is at a level of 1% or more to less than 26%, 10 when it is at a level of 26% or more to less than 51%, 20 when it is at a level of 51 % or more to less than 76%, and 40 when it is at a level of 76% or more to 100% or less, by calculating a percentage based on the value of the negative control group (for example, negative control group infected with protozoa).

In one embodiment, the active ingredient (quinic acid or its salt) may be comprised at 2w/w% or less, 1w/w% or less, less than 1w/w% , $10^{-1}$ w/w% or less, $5 \times 10^{-2}$ w/w% or less, $2.5 \times 10^{-2}$ w/w% or less, $2 \times 10^{-2}$ w/w% or less, $1.25 \times 10^{-2}$ w/w% or less, $10^{-2}$ w/w% or less, $9 \times 10^{-3}$ w/w% or less, $8 \times 10^{-3}$ w/w% or less, $7 \times 10^{-3}$ w/w% or less, $6 \times 10^{-3}$ w/w% or less, $5 \times 10^{3}$ w/w% or less, $4 \times 10^{-3}$ w/w% or less, $10^{-7}$ w/w% or more, $10^{-6}$ w/w% or more, $10^{-5}$ w/w% or more, $10^{-4}$ w/w% or more, $5 \times 10^{-4}$ w/w% or more, $10^{-3}$ w/w% or more, $1.5 \times 10^{-3}$ w/w% or more, $2 \times 10^{-3}$ w/w% or more, $3 \times 10^{-3}$ w/w% or more, $4 \times 10^{-3}$ w/w% or more, $5 \times 10^{-3}$ w/w% or more, $10^{-7}$ to 1w/w%, $10^{-7}$ to $10^{-1}$w/w%, $10^{-7}$ to $5 \times 10^{-2}$ w/w%, $10^{-7}$ to $10^{-2}$ w/w%, $10^{-7}$ to $^{5} \times 10^{-3}$ w/w%, $10^{-7}$ to $4 \times 10^{-3}$ w/w%, $10^{-7}$ to $10^{-3}$ w/w%, $10^{-7}$ to $^{5} \times 10^{-4}$ w/w%, $10^{-7}$ to $10^{-4}$ w/w%, $10^{-7}$ to $10^{-5}$ w/w%, $10^{-6}$ to 1w/w%, $10^{-6}$ to $10^{-1}$w/w%, $10^{-6}$ to $5 \times 10^{-2}$ w/w%, $10^{-6}$ to $10^{-2}$ w/w%, $10^{-6}$ to $5 \times 10^{-3}$ w/w%, $10^{-6}$ to $4 \times 10^{-3}$ w/w%, $10^{-6}$ to $10^{-3}$ w/w%, $10^{-6}$ to $5 \times 10^{-4}$ w/w%, $10^{-6}$ to $10^{-4}$ w/w%, $10^{-6}$ to $10^{-5}$ w/w%, $10^{-5}$ to 1w/w%, $10^{-5}$ to $10^{-1}$w/w%, $10^{-5}$ to $5 \times 10^{-2}$ w/w%, $10^{-5}$ to $10^{-2}$ w/w%, $10^{-5}$ to $5 \times 10^{-3}$ w/w%, $10^{-5}$ to $4 \times 10^{-3}$ w/w%, $10^{-5}$ to $10^{-3}$ w/w%, $10^{-5}$ to $5 \times 10^{-4}$ w/w%, $10^{-5}$ to $10^{-4}$ w/w%, $10^{-4}$ to 1w/w%, $10^{-4}$ to $10^{-1}$w/w%, $10^{-4}$ to $5 \times 10^{-2}$ w/w%, $10^{-4}$ to $10^{-2}$ w/w%, $10^{-4}$ to $5 \times 10^{-3}$ w/w%, $10^{-4}$ to $4 \times 10^{-3}$ w/w%, $10^{-4}$ to $10^{-3}$ w/w%, $10^{-4}$ to $5 \times 10^{-4}$ w/w%, $10^{-3}$ to 1w/w%, $10^{-3}$ to $10^{-1}$w/w%, $10^{-3}$ to $5 \times$

$10^{-2}$ w/w%, $10^{-3}$ to $10^{-2}$ w/w%, $10^{-3}$ to $5 \times 10^{-3}$ w/w%, $10^{-3}$ to $4 \times 10^{-3}$ w/w%, $10^{-3}$ to $2 \times 10^{-3}$ w/w%, or $10^{-3}$ to $1.5 \times 10^{-3}$ w/w% in the feed composition. In one embodiment, the feed composition may be a feed (for example, a mixed feed and/or a single ingredient finally ingested by an animal) comprising the active ingredient within the above range based on the total weight.

In one embodiment, the active ingredient (quinic acid or its salt) may be comprised at a concentration of 10000ppm or less, 5000ppm or less, 2000ppm or less, 1000ppm or less, 500ppm or less, 400ppm or less, 300ppm or less, 250ppm or less, 200ppm or less, 125ppm or less, less than 125ppm, 1 00ppm or less, 90ppm or less, 80ppm or less, 70ppm or less, 65ppm or less, 0.001ppm or more, 0.01ppm or more, 0.1ppm or more, 1ppm or more, 5ppm or more, 10ppm or more, 15ppm or more, 20ppm or more, 30ppm or more, 40ppm or more, 50ppm or more, 0.001 to 2000ppm, 0.001 to 1500ppm, 0.001 to 1000ppm, 0.001 to 500ppm, 0.001 to 300ppm, 0.001 to 200ppm, 0.001 to 125ppm, 0.001 to 100ppm, 0.001 to 90ppm, 0.001 to 80ppm, 0.001 to 70ppm, 0.001 to 60ppm, 0.001 to 50ppm, 0.001 to 40ppm, 0.001 to 30ppm, 0.003 to 2000ppm, 0.003 to 1500ppm, 0.003 to 1000ppm, 0.003 to 500ppm, 0.003 to 300ppm, 0.003 to 200ppm, 0.003 to 125ppm, 0.003 to 100ppm, 0.003 to 90ppm, 0.003 to 80ppm, 0.003 to 70ppm, 0.003 to 60ppm, 0.003 to 50ppm, 0.003 to 40ppm, 0.003 to 30ppm, 0.01 to 2000ppm, 0.01 to 1500ppm, 0.01 to 1000ppm, 0.01 to 500ppm, 0.01 to 300ppm, 0.01 to 200ppm, 0.01 to 125ppm, 0.01 to 100ppm, 0.01 to 90ppm, 0.01 to 80ppm, 0.01 to 70ppm, 0.01 to 60ppm, 0.01 to 50ppm, 0.01 to 40ppm, 0.01 to 30ppm, 0.1 to 2000ppm, 0.1 to 1500ppm, 0.1 to 1000ppm, 0.1 to 500ppm, 0.1 to 300ppm, 0.1 to 200ppm, 0.1 to 125ppm, 0.1 to 100ppm, 0.1 to 90ppm, 0.1 to 80ppm, 0.1 to 70ppm, 0.1 to 60ppm, 0.1 to 50ppm, 0.1 to 40ppm, 0.1 to 30ppm, 1 to 2000ppm, 1 to 1500ppm, 1 to 1000ppm, 1 to 500ppm, 1 to 300ppm, 1 to 200ppm, 1 to 125ppm, 1 to 100ppm, 1 to 90ppm, 1 to 80ppm, 1 to 70ppm, 1 to 60ppm, 1 to 50ppm, 1 to 40ppm, 1 to 30ppm, 3 to 2000ppm, 3 to 1500ppm, 3 to 1000ppm, 3 to 500ppm, 3 to 300ppm, 3 to 200ppm, 3 to 125ppm, 3 to 100ppm, 3 to 90ppm, 3 to 80ppm, 3 to 70ppm, 3 to 60ppm, 3 to 50ppm, 3 to 40ppm, 3 to 30ppm, 5 to 2000ppm, 5 to 1500ppm, 5 to 1000ppm, 5 to 500ppm, 5 to 300ppm, 5 to 200ppm, 5 to 125ppm, 5 to 100ppm, 5 to 90ppm, 5 to 80ppm, 5 to 70ppm, 5 to 60ppm, 5 to 50ppm, 5 to 40ppm, 5 to 30ppm, 10 to 2000ppm, 10 to 1500ppm, 10 to 1000ppm, 10 to 500ppm, 10 to 300ppm, 10 to 200ppm, 10 to 125ppm, 10 to 100ppm, 10 to 90ppm, 10 to 80ppm, 10 to 70ppm, 10 to 60ppm, 10 to 50ppm, 10 to 40ppm or 10 to 30ppm in the feed composition.

In one embodiment, when the active ingredient is comprised in the above range, the anti-coccidial activity may be more excellent.

In the present disclosure, "feed" may mean any natural or artificial diet, meal, or the like or a component of the meal which is for an animal to eat, ingest, and digest, or is suitable therefor. A concentrated feed and/or a special feed may be further comprised in the feed composition according to one embodiment. The concentration feed is a byproduct obtained by refining seed fruits including grains such as wheat, oats, corn and the like, grains and the like, and it may be bran including rice bran, wheat bran, barley bran and the like, sesame dregs which are by-products obtained by extracting soybeans, oil, sesame seeds, linseed, coco palm, and the like, and wastes such as residual starch, which is a main component of starch waste that is the rest after removing starch from sweet potatoes, potatoes, and the like, animal feeds such as fish meal, fish waste, fish soluble that is a concentrated one of fresh liquids obtained from fish, meat meal, blood meal, feather meal, dried whey obtained by drying whey, which is the residue when cheese is prepared from nonfat dry milk and milk, and casein is prepared from skim milk, and the like, yeasts, chlorella, and/or seaweed and the like.

The feed composition according to one embodiment may mean a feed in a form which an animal ingests finally, a dietary supplement which can be mixed in a feed, and/or a feed additive. The dietary supplement may mean, for example, a composition containing a preparation providing a therapeutic agent or digestive agent to an animal, which is a composition that is not a common source of calorie intake for the living body, that is, an energy source, but is added to a common animal feed and ingested. The feed additive, refers to a substance added to a feed on the purpose of various effects such as nutrient supplementation and weight loss prevention, enhancement of digestibility of fiber in a feed, improvement of milk quality, prevention of reproductive disorders and improvement of a conception rate, prevention of high temperature stress in summer, and the like. In one embodiment, it may mean a substance added for the purpose of preventing, alleviating or treating coccidiosis.

In one embodiment, the feed composition may be a feed additive, and when the feed additive according to one embodiment is mixed to a feed (for example, a mixed feed and/or a single ingredient finally ingested by an animal), it may be mixed with feed raw materials, supplementary feeds, supplementary agents, and/or other kinds of additives and the like other than the active ingredient according to one embodiment by being added at a weight of 0.001%(w/w) or more, 0.005%(w/w) or more, 0.01%(w/w) or more, 0.05%(w/w) or more, 0.1%(w/w) or more, 0.5%(w/w) or more, 1%(w/w) or less, 0.5%(w/w) or less, 0.1%(w/w) or less, 0.05%(w/w) or less, 0.01%(w/w) or less, 0.005%(w/w) or less, 0.001 to 1%(w/w), 0.001 to 0.5%(w/w), 0.001 to 0.1%(w/w), 0.001 to 0.05%(w/w), 0.001 to 0.01%(w/w), 0.001 to 0.005%(w/w), 0.005 to 1%(w/w), 0.005 to 0.5%(w/w), 0.005 to 0.1%(w/w), 0.005 to 0.05%(w/w), 0.005 to 0.01%(w/w), 0.01 to 1%(w/w), 0.01 to 0.5%(w/w), 0.01 to 0.1%(w/w), 0.01 to 0.05%(w/w), 0.05 to 1%(w/w), 0.05 to 0.5%(w/w), 0.05 to 0.1%(w/w), 0.1 to 1%(w/w), 0.1 to 0.5%(w/w), or 0.5 to 1%(w/w) based on the total feed weight.

The feed composition may further comprise a commonly used any appropriate excipient or solvent such as water and the like in the feed composition, and this excipient may be for example, a preservative, wetting agent, dispersant,

suspending agent, buffer, stabilizer or tonicifying agent or the like, but not limited thereto.

The pharmaceutical composition according to one embodiment may be used as a single preparation, and may be used by preparing it as a combined preparation by further comprising an authorized pharmaceutical composition known to have a preventive or therapeutic effect for coccidiosis. It may be formulated into a pharmaceutical unit dose form by adding a pharmaceutically acceptable carrier, excipient or diluent.

In the present disclosure, "pharmaceutically acceptable" means that a living organism is not significantly stimulated and biological activity and properties of an administration active substance are not inhibited. The pharmaceutical composition comprising a pharmaceutically acceptable carrier according to one embodiment may have any one formulation selected from the group consisting of tablets, pills, powders, granules, capsules, suspension, oral liquids, emulsion, syrup, sterilized aqueous solution, non-aqueous solvents, suspension, emulsion, lyophilized formulations, and suppositories.

The pharmaceutical composition may be various oral or parenteral formulations. When formulated, it may be prepared by using a commonly used diluent or excipient such as a filler, a thickener, a binder, a wetting agent, a disintegrating agent, or a surfactant.

Solid preparations for oral administration include tablets, pills, powders, granules, capsules and the like, and these solid preparations may be prepared by mixing at least one excipient, for example, starch, calcium carbonate, sucrose or lactose, gelatin, and the like. In addition, in addition to the simple excipient, lubricants such as magnesium stearate, talc and the like may be used. Suspension, oral liquids, emulsion, syrup and the like correspond to liquid preparations for oral administration, and in addition to water and liquid paraffin that are commonly used simple diluents, various excipients, for example, a wetting agent, a sweetener, a flavoring agent, a preservative and the like, may be comprised.

Preparations for parenteral administration may include sterilized aqueous solution, non-aqueous solvents, suspension, emulsion, lyophilized formulations, and suppositories. As the non-aqueous solvents and suspension, propylene glycol, polyethylene glycol, vegetable oils such as olive oil, injectable esters such as ethyl oleate and the like may be used. As the base of the suppositories, witepsol, macrogol, tween 61, cacao butter, laurin butter, glycerogelatin and the like may be used.

In one embodiment, the pharmaceutical composition may be used by formulating into various forms such as oral formulations including powders, granules, tablets, capsules, suspension, emulsion, syrup, aerosols and the like, injections of sterilized injection solution, and the like, and it may be orally administered or administered through various routes including intravenous, intraperitoneal, subcutaneous, intrarectal, local administration and the like.

In one embodiment, the pharmaceutical composition may further comprise a carrier, excipient or diluent or the like additionally, and examples of the appropriate carrier, excipient, or diluent that may be comprised include lactose, dextrose, sucrose, sorbitol, mannitol, xylitol, erythritol, maltitol, starch, acacia gum, alginate, gelatin, calcium phosphate, calcium silicate, cellulose, methyl cellulose, amorphous cellulose, polyvinyl pyrrolidone, water, methyl hydroxy benzoate, propyl hydroxy benzoate, talc, magnesium stearate and mineral oils and the like. In addition, the pharmaceutical composition may further comprise a filler, an anti-coagulant, a lubricant, a wetting agent, a flavoring, an emulsifier, a preservative, and the like additionally.

According to one embodiment, the dose of the active ingredient (quinic acid or its salt) in the pharmaceutical composition may vary depending on a patient (subject)'s age, gender and body weight, and in generally, 0.0001 to 1mg/kg, 0.0001 to 0.1mg/kg, 0.0001 to 0.01mg/kg, 0.0001 to 0.001mg/kg, 0.001 to 1mg/kg, 0.001 to 0.1mg/kg, 0.001 to 0.01mg/kg, 0.01 to 1mg/kg, 0.01 to 0.1mg/kg, 0.1 to 1mg/kg per body weight kg may be administered daily or on alternate days, or be administered by dividing into 1 to 3 times per day. However, it may be increased or reduced depending on the administration route, severity of disease, gender, body weight, age and the like, so the dose does not limit the scope of the present disclosure by any method. In one embodiment, when the composition is intraperitoneally administered, it may be administered at a concentration of 0.001 to 1mg/kg.

In one embodiment, the dose of the pharmaceutical composition may have various ranges depending on a patient's body weight, age, gender, health condition, diet, administration time, administration method, excretion rate and severity of disease and the like.

In one embodiment, the quinic acid or pharmaceutically acceptable salt thereof may be comprised at 2w/w% or less, 1w/w% or less, less than 1w/w%, $10^{-1}$ w/w% or less, $5 \times 10^{-2}$ w/w% or less, $2.5 \times 10^{-2}$ w/w% or less, $2 \times 10^{-2}$ w/w% or less, $1.25 \times 10^{-2}$ w/w% or less, $10^{-2}$ w/w% or less, $9 \times 10^{-3}$ w/w% or less, $8 \times 10^{-3}$ w/w% or less, $7 \times 10^{-3}$ w/w% or less, $6 \times 10^{-3}$ w/w% or less, $5 \times 10^{-3}$ w/w% or less, $4 \times 10^{-3}$ w/w% or less, $10^{-7}$ w/w% or more, $10^{-6}$ w/w% or more, $10^{-5}$ w/w% or more, $10^{-4}$ w/w% or more, $5 \times 10^{-4}$ w/w% or more, $10^{-3}$ w/w% or more, $1.5 \times 10^{-3}$ w/w% or more, $2 \times 10^{-3}$ w/w% or more, $3 \times 10^{-3}$ w/w% or more, $4 \times 10^{-3}$ w/w% or more, $5 \times 10^{-3}$ w/w% or more, $10^{-7}$ to 1w/w%, $10^{-7}$ to $10^{-1}$w/w%, $10^{-7}$ to $5 \times 10^{-2}$ w/w%, $10^{-7}$ to $10^{-2}$ w/w%, $10^{-7}$ to $5 \times 10^{-3}$ w/w%, $10^{-7}$ to $4 \times 10^{-3}$ w/w%, $10^{-7}$ to $10^{-3}$ w/w%, $10^{-7}$ to $5 \times 10^{-4}$ w/w%, $10^{-7}$ to $10^{-4}$ w/w%, $10^{-7}$ to $10^{-5}$ w/w%, $10^{-6}$ to 1w/w%, $10^{-6}$ to $10^{-1}$w/w%, $10^{-6}$ to $5 \times 10^{-2}$ w/w%, $10^{-6}$ to $10^{-2}$ w/w%, $10^{-6}$ to $5 \times 10^{-3}$ w/w%, $10^{-6}$ to $4 \times 10^{-3}$ w/w%, $10^{-6}$ to $10^{-3}$ w/w%, $10^{-6}$ to $5 \times 10^{-4}$ w/w%, $10^{-6}$ to $10^{-4}$ w/w%, $10^{-6}$ to $10^{-5}$ w/w%, $10^{-5}$ to 1w/w%, $10^{-5}$ to $10^{-1}$w/w%, $10^{-5}$ to $5 \times 10^{-2}$ w/w%, $10^{-5}$ to $10^{-2}$ w/w%, $10^{-5}$ to $5 \times 10^{-3}$ w/w%, $10^{-5}$ to $4 \times 10^{-3}$ w/w%, $10^{-5}$ to

$10^{-3}$ w/w%, $10^{-5}$ to $5 \times 10^{-4}$ w/w%, $10^{-5}$ to $10^{-4}$ w/w%, $10^{-4}$ to 1w/w%, $10^{-4}$ to $10^{-1}$w/w%, $10^{-4}$ to $5 \times 10^{-2}$ w/w%, $10^{-4}$ to $10^{-2}$ w/w%, $10^{-4}$ to $5 \times 10^{-3}$ w/w%, $10^{-4}$ to $4 \times 10^{-3}$ w/w%, $10^{-4}$ to $10^{-3}$ w/w%, $10^{-4}$ to $5 \times 10^{-4}$ w/w%, $10^{-3}$ to 1w/w%, $10^{-3}$ to $10^{-1}$w/w%, $10^{-3}$ to $5 \times 10^{-2}$ w/w%, $10^{-3}$ to $10^{-2}$ w/w%, $10^{-3}$ to $5 \times 10^{-3}$ w/w%, $10^{-3}$ to $4 \times 10^{-3}$ w/w%, $10^{-3}$ to $2 \times 10^{-3}$ w/w%, or $10^{-3}$ to $1.5 \times 10^{-3}$ w/w% in the pharmaceutical composition.

According to one embodiment, the composition may be administered in a pharmaceutically effective dose. In the present disclosure, 'pharmaceutically effective dose' may mean an amount sufficient for treating a disease at a reasonable benefit/risk ratio applicable for medical treatment, and the effective dose level may be determined depending on factors including a patient's disease type, severity, drug activity, sensitivity to drug, administration time, an administration route, and an excretion rate, a treatment period, and a concurrently used drug and other factors well known in the medical field. According to one embodiment, the composition may be administered as an individual therapeutic agent, or may be administered with other anti-coccidial agents in combination, and may be administered with a conventional therapeutic agent simultaneously, separately, or sequentially, and may be administered single or multiple times. It is important to administer an amount which can obtain a maximal effect with a minimal amount without side effects in consideration of all the elements, and this may be easily determined by those skilled in the art.

In one embodiment, the quinic acid or its pharmaceutically acceptable salt may be comprised in the pharmaceutical composition at a concentration of 10000ppm or less, 5000ppm or less, 2000ppm or less, 1000ppm or less, 500ppm or less, 400ppm or less, 300ppm or less, 250ppm or less, 200ppm or less, 125ppm or less, less than 125ppm, 100ppm or less, 90ppm or less, 80ppm or less, 70ppm or less, 65ppm or less, 0.001ppm or more, 0.01ppm or more, 0.1ppm or more, 1ppm or more, 5ppm or more, 10ppm or more, 15ppm or more, 20ppm or more, 30ppm or more, 40ppm or more, 50ppm or more, 0.001 to 2000ppm, 0.001 to 1500ppm, 0.001 to 1000ppm, 0.001 to 500ppm, 0.001 to 300ppm, 0.001 to 200ppm, 0.001 to 125ppm, 0.001 to 100ppm, 0.001 to 90ppm, 0.001 to 80ppm, 0.001 to 70ppm, 0.001 to 60ppm, 0.001 to 50ppm, 0.001 to 40ppm, 0.001 to 30ppm, 0.003 to 2000ppm, 0.003 to 1500ppm, 0.003 to 1000ppm, 0.003 to 500ppm, 0.003 to 300ppm, 0.003 to 200ppm, 0.003 to 125ppm, 0.003 to 100ppm, 0.003 to 90ppm, 0.003 to 80ppm, 0.003 to 70ppm, 0.003 to 60ppm, 0.003 to 50ppm, 0.003 to 40ppm, 0.003 to 30ppm, 0.01 to 2000ppm, 0.01 to 1500ppm, 0.01 to 1000ppm, 0.01 to 500ppm, 0.01 to 300ppm, 0.01 to 200ppm, 0.01 to 125ppm, 0.01 to 100ppm, 0.01 to 90ppm, 0.01 to 80ppm, 0.01 to 70ppm, 0.01 to 60ppm, 0.01 to 50ppm, 0.01 to 40ppm, 0.01 to 30ppm, 0.1 to 2000ppm, 0.1 to 1500ppm, 0.1 to 1000ppm, 0.1 to 500ppm, 0.1 to 300ppm, 0.1 to 200ppm, 0.1 to 125ppm, 0.1 to 100ppm, 0.1 to 90ppm, 0.1 to 80ppm, 0.1 to 70ppm, 0.1 to 60ppm, 0.1 to 50ppm, 0.1 to 40ppm, 0.1 to 30ppm, 1 to 2000ppm, 1 to 1500ppm, 1 to 1000ppm, 1 to 500ppm, 1 to 300ppm, 1 to 200ppm, 1 to 125ppm, 1 to 100ppm, 1 to 90ppm, 1 to 80ppm, 1 to 70ppm, 1 to 60ppm, 1 to 50ppm, 1 to 40ppm, 1 to 30ppm, 3 to 2000ppm, 3 to 1500ppm, 3 to 1000ppm, 3 to 500ppm, 3 to 300ppm, 3 to 200ppm, 3 to 125ppm, 3 to 100ppm, 3 to 90ppm, 3 to 80ppm, 3 to 70ppm, 3 to 60ppm, 3 to 50ppm, 3 to 40ppm, 3 to 30ppm, 5 to 2000ppm, 5 to 1500ppm, 5 to 1000ppm, 5 to 500ppm, 5 to 300ppm, 5 to 200ppm, 5 to 125ppm, 5 to 100ppm, 5 to 90ppm, 5 to 80ppm, 5 to 70ppm, 5 to 60ppm, 5 to 50ppm, 5 to 40ppm, 5 to 30ppm, 10 to 2000ppm, 10 to 1500ppm, 10 to 1000ppm, 10 to 500ppm, 10 to 300ppm, 10 to 200ppm, 10 to 125ppm, 10 to 100ppm, 10 to 90ppm, 10 to 80ppm, 10 to 70ppm, 10 to 60ppm, 10 to 50ppm, 10 to 40ppm or 10 to 30ppm.

In one embodiment, the pharmaceutical composition may be administered into a subject through various routes. The administration may mean providing a certain substance for a subject (patient) by any appropriate method, and the administration route of the pharmaceutical composition may be oral administration and/or parenteral administration through all general routes as long as it can reach target tissue. In case of parenteral administration, skin external application, intraperitoneal injection, intrarectal injection, subcutaneous injection, intravenous injection, intramuscular injection, and/or intrathoracic injection may be selected. In addition, the composition according to one embodiment may be administered by using any device capable of deliver the active ingredient into target cells.

In one embodiment, the subject to which the composition or method is applied means an animal in which coccidiosis was developed or may be developed, and the animal may be a mammal including humans, horses, cattle, mice, rats, dogs, cats and the like, birds including poultry (for example, breeders, broilers, and/or laying hens, etc.) and the like, fish, amphibians, and/or reptiles and the like.

In one embodiment, the animal to which the composition or method is applied may be at least one selected from the group consisting of the animals described in Table 1 above, and for example, it may be at least one selected from the group consisting of humans, chickens, ducks, geese, turkeys, quails, pheasants, pigeons, parrots, cattle, pigs, goats, sheep, horses, antelopes, oryxes, monkeys, cats, dogs, mice, rats, rabbits, racoons, squirrels, bats, guinea pigs, camels, llamas, alpacas, wombats, lizards, goldfish, crucian carps, tilapia, barbel, lungfish and European chub. In one embodiment, the animal may be an animal except for humans.

Other aspect provides an anti-protozoal composition against *Eimeria* sp. protozoa comprising quinic acid and/or its salt.

Other aspect provides a use of quinic acid and/or its salt for using in preparation of an anti-protozoal composition against *Eimeria* sp. protozoa.

Other aspect provides a method for controlling *Eimeria* sp. protozoa, comprising administering quinic acid and/or its salt into a subject in need of controlling *Eimeria* sp. protozoa.

Matters of the quinic acid and *Eimeria* sp. protozoa are as described above.

The anti-protozoal composition may further comprise any appropriate excipient or solvent such as water commonly used in an anti-protozoal composition, and this excipient may be for example, a preservative, wetting agent, dispersant, suspending agent, buffer, stabilizer or tonicifying agent or the like, but not limited thereto.

In one embodiment, that the anti-protozoal activity (effect, efficacy) against *Eimeria* sp. protozoa is excellent may mean the following (1) and/or (2) characteristics, and for example, it may exhibit the following (1) and/or (2) characteristics compared to a control group (negative control group and/or positive control group):

(1) Excellent killing effect of *Eimeria* sp. protozoa; and/or
(2) Inhibition of a cell penetration effect of *Eimeria* sp. protozoa and/or a proliferation effect of the protozoa in cells.

In one embodiment, the quinic acid or its salt may be comprised at the afore-mentioned concentration range in the anti-protozoal composition in the feed composition and/or pharmaceutical composition.

Other aspect provides a feed additive comprising an anti-protozoal composition against *Eimeria* sp. protozoa.

Other aspect provides a feed comprising the feed additive.

The feed may be prepared by separately preparing the anti-protozoal composition in a feed additive form and mixing it in the feed, or directly adding it during preparation of the feed.

The anti-protozoal composition in the feed may be in a liquid or dry state, and for example, it may be a dried powder form. The anti-protozoal composition may be comprised in an amount of 0.005 to 10 % by weight, 0.05 to 10 % by weight, 0.1 to 10 % by weight, 0.005 to 5 % by weight, 0.05 to 5 % by weight, 0.1 to 5 % by weight, 0.005 to 2 % by weight, 0.05 to 2 % by weight, or 0.1 to 2 % by weight of the total feed weight, but not limited thereto. In addition, the feed may further comprise common additives which can increase preservability of the feed in addition to the anti-protozoal composition.

In the present description, the feed to which the anti-protozoal composition can be added may be selected from the group consisting of commercially available feed or grains, roots and fruits, food processing by-products, algae, fibers, pharmaceutical by-products, fats and oils, starches, meal, grain by-products, proteins, inorganic materials, fats and oils, minerals, single cell proteins, zooplankton, leftover foods, and the like, but not limited thereto.

Other embodiment provides a food additive or a drinking water additive comprising the anti-protozoal composition as an active ingredient. By supplying the anti-protozoal composition by mixing it in drinking water, the number of the *Eimeria* sp. protozoa in the drinking water can be reduced. The *Eimeria* sp. protozoa are as described above.

Other aspect provides a disinfectant comprising the anti-protozoal composition.

Other aspect provides a method for disinfection, comprising applying the anti-protozoal composition into a subject in need of disinfection. The disinfectant collectively refers to an agent which can control *Eimeria* sp. protozoa, and it may be used for general household disinfectants, disinfectants of food and cooking areas and equipment, and disinfection of various kinds of growing supplies such as buildings such as poultry farms, livestock sheds, and the like, livestock, drinking water, litter, egg trays, transport vehicles, tableware and the like.

Other aspect provides a detergent comprising the anti-protozoal composition.

Other aspect provides a method for washing, comprising applying the anti-protozoal composition into a subject in need of washing of *Eimeria* sp. protozoa.

As the anti-protozoal composition has a killing effect against *Eimeria* sp. protozoa, it may be applied as a use of washing (cleaning) the skin surface or each part of the body and the like of a subject who has been exposed or is likely to be exposed to *Eimeria* sp. protozoa.

[ADVANTAGEOUS EFFECTS]

The present disclosure relates to a composition for preventing, alleviating or treating coccidiosis comprising quinic acid, and an anti-protozoal composition against *Eimeria* sp. protozoa, and has an excellent killing effect against *Eimeria* sp. protozoa.

[BRIEF DESCRIPTION OF THE DRAWINGS]

FIG. 1 shows the results of treating *Eimeria tenella* protozoa with various concentrations of quinic acid and measuring the mortality rate of the protozoa.

[MODE FOR INVENTION]

Hereinafter, the present invention will be described in more detail by the following examples. However, these are intended to illustrate the present invention only, but the scope of the present invention is not limited by these examples.

**Example 1. Direct killing effect against *Eimeria*** protozoa

A certain amount of oocysts (eggs) of *Eimeria tenella* and *Eimeria maxima* protozoa (College of Veterinary Medicine, Gyeongsang National University, Korea) were put in a tube containing glass beads and crushed, and then, in order to remove crushed oocyst cell walls and other debris, internal sporocysts were purified using Percoll density gradient, and washed with a PBS solution. For excystation of internal sporozoites, reagents comprising sodium taurocholic acid (sigma aldirich, USA) and trypsin (Ginco, USA), respectively, were treated to *Eimeria tenella* and *Eimeria maxima* sporangia and they were incubated, and then washed with PBS (Phosphate-Buffered Saline) solution once and the protozoa were obtained.

**Example 1-1. Direct Killing Effect against Eimeria sp. protozoa of Quinic Acid by concentration**

Quinic acid (Sigma, Cas No. 77-95-2) at concentrations ranging from 0 to 1000 ppm was reacted with *Eimeria tenella* oocysts at 40°C for 3 hours. Following this, only live protozoa (sporozoites) were counted by microscopy. The mortality rate (%) of the protozoa was measured for each substance treatment compared to the negative control group treated with PBS. The results, as shown in Figure 1 and Table 2, confirmed the anti-coccidial efficacy of quinic acid depending on the concentration.

[Table 2]

| The amount of Quinic acid (ppm) | 1000 | 500 | 250 | 100 | 50 | 25 | 12.5 | 6.25 | 3.12 5 | 1.56 | 0.78 |
|---|---|---|---|---|---|---|---|---|---|---|---|
| The mortality rate | 100. 0% | 97.9 % | 91.5 % | 59.6 % | 55.3 % | 35.1 % | 31.9 % | 25.5 % | 20.2 % | 14.9 % | 7.4 % |

**Example 1-2. Comparison of efficacy among chlorogenic acid components**

The killing effect against *Eimeria* sp. protozoa was confirmed for quinic acid (sigma, Cas No. 77-95-2), chlorogenic acid (sigma, Cas No. 327-97-9), m-coumaric acid (sigma, Cas No. 14755-02-3), caffeic acid (sigma, Cas No. 331-39-5), and ferulic acid (sigma, Cas No. 1135-24-6).

After reacting 0.1%(v/v) DMSO, quinic acid, chlorogenic acid, m-coumaric acid, caffeic acid and ferulic acid, respectively, with *Eimeria tenella* and *Eimeria maxima* protozoa at a concentration of 10 ppm at 40°C for 3 hours, only living protozoa (sporozoites) were counted by microscopy. Then, the mortality rate (%) was measured when each substance was treated compared to a negative control group to which PBS was treated, and the result is shown in Table 3 below.

[Table 3]

| sample | Dose (ppm) | Mortality rate of *Eimeria tenella* orozites [Killed sporozoites %] | Mortality rate of *Eimeria maxima* orozites [Killed sporozoites %] |
|---|---|---|---|
| Cell only (PBS treated, negative control group) | - | 0 | 0 |
| 0.1% DMSO | - | 0 | 0 |
| Quinic acid | 10 | 32 | 34 |
| Chlorogenic acid | 10 | 0 | 0 |
| m-coumaric acid | 10 | 10.7 | 6.4 |
| Caffeic acid | 10 | 0 | 0 |
| Ferulic acid | 10 | 0 | 0 |

As shown in Table 3 above, quinic acid showed a superior direct killing effect against *Eimeria tenella* and *Eimeria maxima* protozoa to chlorogenic acid, m-coumaric acid, caffeic acid, and ferulic acid at the same concentration (10 ppm).

In addition, the *Eimeria tenella* protozoa were killed by treating each substance up to 1000 ppm concentration, and the minimum treatment concentration of each substance at which *Eimeria tenella* protozoa showed a mortality rate of 50% or higher is shown in Table 4 below.

[Table 4]

| sample | Minimum Dose for killed 50% sporozoite (ppm) |
|---|---|
| Quinic acid | 50 |
| Chlorogenic acid | 500 |
| m-coumaric acid | 500 |
| Caffeic acid | X |
| Ferulic acid | X |

As shown in Table 4 above, it was confirmed that quinic acid has a significantly superior the killing effect against *Eimeria tenella* protozoa, as the minimum concentration of quinic acid showing a mortality rate of 50% or higher against *Eimeria tenella* protozoa was lower than chlorogenic acid, m-coumaric acid, caffeic acid, and ferulic acid.

**Example 1-3. Comparison of Efficacy among Chlorogenic acid Family**

The killing effect against *Eimeria* sp. protozoa was confirmed for quinic acid (sigma, Cas No. 77-95-2), chlorogenic acid (sigma, Cas No. 327-97-9), theogallin (3-galloylquinic acid, sigma, Cas No.17365-11-6), and cryptochlorogenic acid (sigma, Cas No. 905-99-7).

After reacting 0.1%(v/v) DMSO, quinic acid, chlorogenic acid, theogallin, and cryptochlorogenic acid, respectively, with *Eimeria tenella* protozoa at a concentration of 10 ppm at 40°C for 3 hours, only living protozoa (sporozoites) were counted by microscopy. Then, the mortality rate (%) was measured when each substance was treated compared to a negative control group to which PBS was treated, and the result is shown in Table 5 below.

[Table 5]

| sample | Dose (ppm) | Mortality rate of *Eimeria tenella* sporozites [Killed sporozoites %] |
|---|---|---|
| Cell only (PBS treated, negative control group) | - | 0 |
| 0.1% DMSO | - | 0 |
| Quinic acid | 10 | 32 |
| Chlorogenic acid | 10 | 0 |
| Theogallin | 10 | 0 |
| Cryptochlorogenic acid | 10 | 0 |

As shown in Table 5 above, quinic acid showed a superior direct killing effect against *Eimeria tenella* protozoa to chlorogenic acid, theogallin, and cryptochlorogenic acid at the same concentration (10 ppm).

**Example 1-4. Comparison of efficacy compared to a sugarcane extract**

The killing effect against *Eimeria* sp. protozoa was confirmed for quinic acid and a sugarcane extract. Sugarcanes from China, Vietnam and Brazil were used, and sugarcane extracts was prepared by using water, methanol and organic solvents (3: 1: 1 mixture based on the volume of methanol: water: chloroform) as an extraction solvent.

The *Eimeria tenella* protozoa were killed by treating the quinic acid or sugarcane extract up to 1000 ppm concentration at maximum, and the minimal treatment concentration showing a mortality rate of 50% or more against *Eimeria tenella* protozoa was shown in Table 6 below.

[Table 6]

| sample | Country of origin | Extraction method | Minimum Dose for killed 50% sporozoite (ppm) |
|---|---|---|---|
| Quinic acid | - | - | 50 |
| Sugarcane extract | China | water | X |

(continued)

| sample | Country of origin | Extraction method | Minimum Dose for killed 50% sporozoite (ppm) |
|---|---|---|---|
| Sugarcane extract | China | methanol | X |
| Sugarcane extract | China | 3:1:1 mixture of methanol/water/$CHCl_3$ (volume basis) | X |
| Sugarcane extract | Vietnam | water | X |
| Sugarcane extract | Vietnam | methanol | X |
| Sugarcane extract | Vietnam | 3:1:1 mixture of methanol/water/$CHCl_3$ (volume basis) | X |
| Sugarcane extract | Brazil | water | X |
| Sugarcane extract | Brazil | methanol | X |
| Sugarcane extract | Brazil | 3:1:1 mixture of methanol/water/$CHCl_3$ (volume basis) | X |

As shown in Table 6 above, it was confirmed that quinic acid has a significantly superior the killing effect against *Eimeria tenella* protozoa, as the sugarcane extract obtained by various origins and extraction methods did not show the killing effect against *Eimeria tenella* protozoa up to 1000 ppm concentration at maximum.

From the above description, those skilled in the art to which the present invention pertains will understand that the present invention can be implemented in other specific forms without changing its technical spirit or essential features. In this regard, the examples described above should be understood as illustrative and not restrictive in all aspects. The scope of the present invention should be interpreted as all changed or modified forms derived from the meaning and scope of the claims described below and equivalent concepts thereof are included in the scope of the present invention.

## Claims

1. A feed composition for preventing or alleviating coccidiosis, comprising a quinic acid or a salt thereof as an active ingredient.

2. The feed composition according to claim 1, wherein the coccidiosis is induced by an *Eimeria* sp. protozoan.

3. The feed composition according to claim 2, wherein the *Eimeria* sp. protozoan is *Eimeria tenella* or *Eimeria maxima.*

4. The feed composition according to claim 1, wherein the feed composition is a feed comprising the active ingredient at a concentration of 1% (w/w) or less based on the total weight.

5. The feed composition according to any one of claims 1 to 4, wherein the feed composition is a feed additive.

6. A pharmaceutical composition for preventing or treating coccidiosis, comprising a quinic acid or a salt thereof as an active ingredient.

7. The pharmaceutical composition according to claim 6, wherein the coccidiosis is induced by an *Eimeria* sp. protozoan.

8. The pharmaceutical composition according to claim 7, wherein the *Eimeria* sp. protozoan is *Eimeria tenella* or *Eimeria maxima.*

9. An antiprotozoal composition against an *Eimeria* sp. protozoan, comprising a quinic acid or a salt thereof as an active ingredient.

10. The antiprotozoal composition according to claim 9, wherein the *Eimeria* sp. protozoan is *Eimeria tenella* or *Eimeria maxima.*

11. A feed additive, comprising the antiprotozoal composition according to claim 9 or 10.

12. A disinfectant, comprising the antiprotozoal composition according to claim 9 or 10.

**13.** A detergent, comprising the antiprotozoal composition according to claim 9 or 10.

**14.** A method for preventing, alleviating or treating coccidiosis, comprising a step of administering a composition selected from the group consisting of the feed composition according to any one of claims 1 to 4, the pharmaceutical composition according to any one of claims 6 to 8, and the antiprotozoal composition according to claim 9 or 10 to an animal except for human.

【FIG. 1】

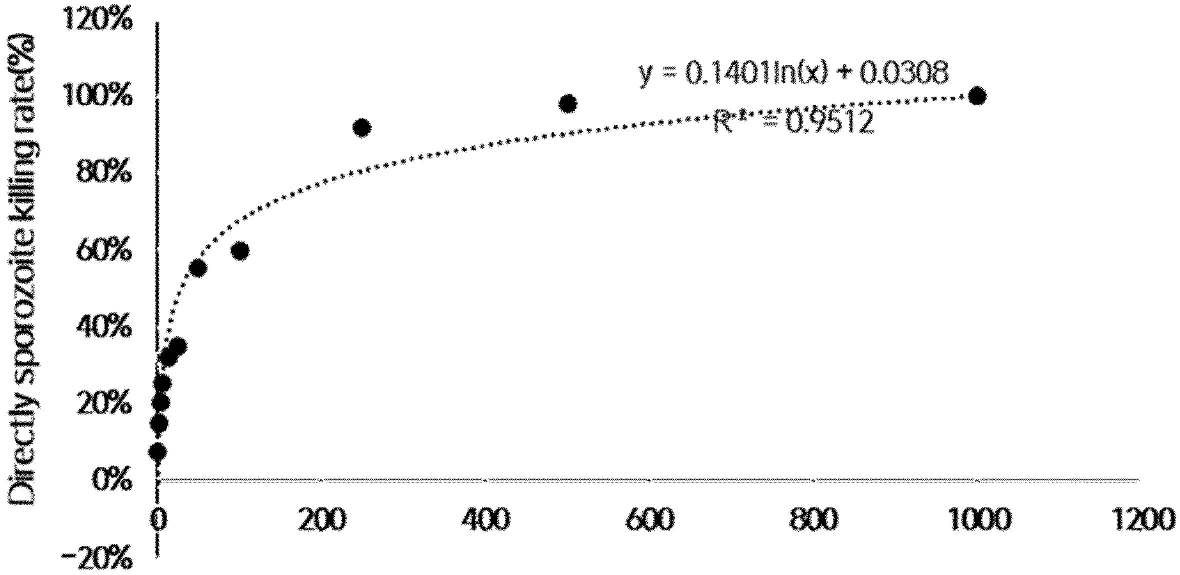
Directly sporozoite killing rate(%)
120%
100%
80%
60%
40%
20%
0%
-20%
0
200
400
600
800
1000
1200
y = 0.1401ln(x) + 0.0308
R² = 0.9512

**INTERNATIONAL SEARCH REPORT**

International application No.
**PCT/KR2024/011650**

**A. CLASSIFICATION OF SUBJECT MATTER**

**A23K 20/105**(2016.01)i; **A61K 31/19**(2006.01)i; **A61P 33/02**(2006.01)i; **A01N 37/02**(2006.01)i; **A01P 1/00**(2006.01)i; **C11D 3/20**(2006.01)i

According to International Patent Classification (IPC) or to both national classification and IPC

**B. FIELDS SEARCHED**

Minimum documentation searched (classification system followed by classification symbols)

A23K 20/105(2016.01); A23K 10/30(2016.01); A23K 20/10(2016.01); A23K 20/111(2016.01); A23K 50/00(2016.01); A61K 35/747(2015.01); A61P 33/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 콕시듐증(coccidiosis), 아이메리아 속(Eimeria spp.), 퀸산(quinic acid), 사료(feed)

**C. DOCUMENTS CONSIDERED TO BE RELEVANT**

| Category* | Citation of document, with indication, where appropriate, of the relevant passages | Relevant to claim No. |
|---|---|---|
| X | DAS, Q. et al. Gut Microbiota, Blood Metabolites, and Spleen Immunity in Broiler Chickens Fed Berry Pomaces and Phenolic-Enriched Extractives. Frontiers in Veterinary Science. 2020, vol. 7, document no. 150, pp. 1-19. See pages 1-3, 5-7 and 15-16; and table 4. | 1-14 |
| A | KR 10-2022-0125628 A (CJ CHEILJEDANG CORPORATION) 14 September 2022 (2022-09-14) See claims 1-9. | 1-14 |
| A | KR 10-2018-0083818 A (CHA, Jang Ock) 23 July 2018 (2018-07-23) See claims 1-12. | 1-14 |
| A | CN 111297913 A (QINGDAO AGRICULTURAL UNIVERSITY) 19 June 2020 (2020-06-19) See claims 1-9. | 1-14 |
| A | KR 10-2020-0012969 A (MOOTRAL SA) 05 February 2020 (2020-02-05) See claims 1-32. | 1-14 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 November 2024** | **11 November 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/011650**

| Patent document cited in search report | Publication date (day/month/year) | Patent family member(s) | Publication date (day/month/year) |
|---|---|---|---|
| KR 10-2022-0125628 A | 14 September 2022 | CN 116528847 A | 01 August 2023 |
| | | EP 4302608 A1 | 10 January 2024 |
| | | JP 2023-545650 A | 31 October 2023 |
| | | US 2024-0299324 A1 | 12 September 2024 |
| | | WO 2022-186648 A1 | 09 September 2022 |
| KR 10-2018-0083818 A | 23 July 2018 | KR 10-2106477 B1 | 06 May 2020 |
| | | WO 2018-131930 A1 | 19 July 2018 |
| CN 111297913 A | 19 June 2020 | CN 111297913 B | 11 October 2022 |
| KR 10-2020-0012969 A | 05 February 2020 | CN 110708965 A | 17 January 2020 |
| | | CN 110944517 A | 31 March 2020 |
| | | CN 110944517 B | 04 June 2024 |
| | | EP 3629756 A1 | 08 April 2020 |
| | | EP 3629757 A1 | 08 April 2020 |
| | | JP 2020-521500 A | 27 July 2020 |
| | | JP 2020-521503 A | 27 July 2020 |
| | | JP 2023-012475 A | 25 January 2023 |
| | | KR 10-2020-0012970 A | 05 February 2020 |
| | | US 12053003 B2 | 06 August 2024 |
| | | US 2020-0138056 A1 | 07 May 2020 |
| | | US 2020-0170281 A1 | 04 June 2020 |
| | | WO 2018-220338 A1 | 06 December 2018 |
| | | WO 2018-220340 A1 | 06 December 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020230103638
- US 20030091589 A1


### Non-patent literature cited in the description


- **WILLIAMS RB**. A compartmentalised model for the estimation of the cost of coccidiosis to the world's chicken production industry. *Int J Parasitol.*, 1999, vol. 29 (8), 1209-1229
- *CHEMICAL ABSTRACTS*, 77-95-2
- **JOHNSON JK** ; **REID WM**. Joyce Johnson, W.Malcolm Reid, Anticoccidial drugs: Lesion scoring techniques in battery and floor-pen experiments with chickens. *Experimental parasitology*, 1970
- *CHEMICAL ABSTRACTS*, 327-97-9
- *CHEMICAL ABSTRACTS*, 14755-02-3
- *CHEMICAL ABSTRACTS*, 331-39-5
- *CHEMICAL ABSTRACTS*, 1135-24-6
- *CHEMICAL ABSTRACTS*, 17365-11-6
- *CHEMICAL ABSTRACTS*, 905-99-7